# EUROPEAN PATENT APPLICATION

(11) **EP 1 172 104 A1**
(43) Date of publication of application: **16.01.2002**
(21) Application number: 00903976.9
(22) Date of filing: 16.02.2000
(51) Int. Cl.: A61K 31/343, A61K 9/48, A61K 47/44, A61P 9/10

(54) **SEAM SOFT CAPSULE PREPARATIONS CONTAINING DIHYDROBENZOFURAN DERIVATIVE**

(30) Priority: 23.02.1999 JP 4520899
(71) Applicant: CHUGAI SEIYAKU KABUSHIKI KAISHA, Tokyo, 115-8543 (JP)
(72) Inventor: KATSUKI, Hisakazu, Chugai Seiyaku Kabushiki Kaisha, Tokyo 171-8545 (JP)
(74) Representative: VOSSIUS & PARTNER
(86) International application number: JP0000862
(87) International publication number: WO0050029

(57) **Abstract**

A seamed soft capsule formulation in which a solution of BO-653, 4,6-di-tert-butyl-2,2-di-n-pentyl-5-hydroxy-2,3-dihydrobenzofuran, in a fat and oil is filled into a seamed soft capsule is provided. This BO-653 formulation confers an excellent stability and a capability of being absorbed by body to BO-653.

## Description

### TECHNICAL FIELD

The present invention relates to a formulation containing a 5-acetoxy or hydroxy-4,6-di-tert-butyl-2,3-dihydrobenzofuran derivative, especially BO-653, and having an excellent stability and a capability of being absorbed by body.

### BACKGROUND ART

A family of 5-acetoxy or hydroxy-4,6-di-tert-butyl-2,3-dihydrobenzofuran derivatives including BO-653, i.e., 4,6-di-tert-butyl-2,2-di-n-pentyl-5-hydroxy-2,3-dihydrobenzofuran, are antioxidative compounds, which are generally oily and high-viscous, and thus promise to be useful for treating an arteriosclerosis and the like (WO94/08930). Generally, if such a compound is used as a drug substance for preparing a formulation, it will be required to confer a high stability, a capability of being absorbed by body and the like to the compound.

For example, as a method for preventing the oxidation of a compound such as vitamin E, which is known as a natural antioxidant, it has been reported that the compound is combined with another antioxidant or dissolved in a fat and oil such as unsaturated higher fatty acid having double bond(s) and then filled into a soft capsule (JP 7-58082A/95 and JP 8-175967A/96, respectively).

On the other hand, as a method for improving the capability of being absorbed by body of a drug which is highly fat-soluble and slightly water-soluble, it has been reported that the drug is combined with a solubilizer such as a non-ionic surfactant to enhance its water-solubility (JP 7-242535A/95) or is dissolved in a fat and oil such as a medium chain fatty acid triglyceride (JP 8-92088A/96) or a higher unsaturated fatty acid (JP 7-258082A/95).

However, since it is common that a structural difference between compounds is responsible for any differences between them in chemical, physical and/or biological properties, these methods would not be applied to the above-mentioned dihydrobenzofuran derivative, especially BO-653.

Therefore, it would be desirable to provide a formulation which specifically confers a high stability and a capability of being absorbed by body to the derivative.

### DISCLOSURE OF THE INVENTION

An object of the present invention is to provide a formulation containing a 5-acetoxy or hydroxy-4,6-di-tert-butyl-2,3-dihydrobenzofuran derivative, especially BO-653, or probucol which formulation confers a high stability and a capability of being absorbed by body to the derivative or probucol.

As a result of extensive research, we found that, when the dihydrobenzofuran derivative or probucol is dissolved in a fat and oil without any additional antioxidant and then filled into a seamed soft capsule, not only its stability is maintained but also its capability of being absorbed by body is improved.

Accordingly, the present invention provides a seamed soft capsule formulation in which a solution of dihydrobenzofuran derivative represented by general formula (1): 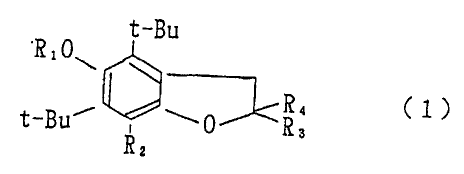 wherein R₁ represents a hydrogen atom or an acyl group; R₂ represents a hydrogen atom or a lower alkyl group; and R₃ and R₄, which may be identical or different, represent a hydrogen atom or a substituted or unsubstituted alkyl, alkenyl, alkynyl or aryl group, or probucol in a fat and oil, is filled into a seamed soft capsule.

The present invention also provides a seamed soft capsule formulation containing BO-653. More specifically, the present invention provides a seamed soft capsule formulation in which a solution of BO-653 in fat and oil is filled into a seamed soft capsule.

### BRIEF DESCRIPTION OF THE DRAWINGS

Fig. 1 shows the influence of oxygen on the stability of BO-653 in various fat and oils.
Fig. 2 shows plasma BO-653 levels after a solution of BO-653 in various solvents is orally administered.

### PREFERRED EMBODIMENTS OF THE INVENTION

The drug substance of the formulation according to the present invention is a 5-acetoxy or hydroxy-4,6-di-tert-butyl-2,3-dihydrobenzofuran derivative represented by the above general formula (1), which is known as described in WO94/08930, or probucol. BO-653 is preferable. As used herein, BO-653 refers to 4,6-di-tert-butyl-2,2-di-n-pentyl-5-hydroxy-2,3-dihydrobenzofuran.

In the definition of the compounds of general formula (1), the examples of the acyl group represented by R₁ include acetyl, formyl, propionyl, benzoyl and benzyloxycarbonyl groups.

The lower alkyl group represented by R₂ means a straight or branched alkyl group having 1 to 6 carbon atoms, the examples of which include, for example, methyl, ethyl, n-propyl, i-propyl, n-butyl, s-butyl and t-butyl groups.

The alkyl group represented by R₃ and R₄ means a straight or branched alkyl group having 1 to 20 carbon atoms, the examples of which include, for example, methyl, ethyl, n-propyl, i-propyl, n-butyl, s-butyl, t-butyl, pentyl, hexyl, heptyl, octyl, nonyl and decyl groups.

The alkenyl group represented by R₃ and R₄ means a straight or branched alkenyl group having 2 to 20 carbon atoms, the examples of which include, for example, vinyl, allyl, butenyl, pentenyl, geranyl and farnesyl groups.

The alkynyl group represented by R₃ and R₄ means a straight or branched alkynyl group having 2 to 20 carbon atoms, the examples of which include, for example, ethynyl, propynyl and butynyl groups.

The aryl group represented by R₃ and R₄ means a monovalent aromatic hydrocarbon formed by removing one hydrogen atom from the aromatic hydrocarbon, the examples of which include, for example, phenyl, tolyl, xylyl, biphenyl, naphthyl, anthryl and phenanthryl groups. The ring carbon atoms of the aryl group may be substituted by one or more groups selected from halogen, lower alkyl, hydroxyl, alkoxy, amino, nitro or trifluoromethyl.

The examples of substituents on these alkyl, alkenyl, alkynyl and aryl groups represented by R₃ and R₄ include halogen, hydroxyl, amino, alkoxy and aryloxy.

The examples of the fat and oil according to the present invention include vegetable oils, medium chain fatty acid triglycerides (hereinafter referred to as MCTs), tricaprylin, oleic acid, linoleic acid, linolenic acid and lecithin. Vegetable oils are preferable.

Preferred vegetable oils are those containing an unsaturated fatty acid as a main component, the examples of which include, for example, soybean oil, safflower oil, sunflower oil, corn oil, cottonseed oil, sesame oil, rapeseed oil, peanut oil and olive oil, among which soybean oil is especially preferred. As used herein, the term "unsaturated fatty acid" means a fatty acid having one or more double bonds in its molecule, the examples of which include, for example, oleic acid, linoleic acid and linolenic acid. To be regarded as the main component, the unsaturated fatty acid should be present in the vegetable oil in an amount of 50% or more of the total amount of the vegetable oil.

The amount of the fat and oil which may be present in the formulation of this invention is not limited so far as it enables the derivative or probucol to be formulated with the fat and oil. However, the fat and oil may be present in the formulation in an amount of 10-98% by weight based on the total weight of the filling solution.

As used herein, the term "soft capsule formulation" means a formulation in which a solution of a drug in a fat and oil is encapsulated in a capsule shell made of a gelatin-based material. The term "seamed soft capsule" , as used herein, means the soft capsule which is prepared by a rotary method. The rotary method is for producing a soft capsule and comprises the steps of stamping capsule shells out of a gelatin sheet which has been formed by gelling a solution containing gelatin, plasticizer and purified water, and then fusing the edges of the capsule shells each other with injecting filling solution therein to form a soft capsule. Hence, this method is also called "stamping method". The formed soft capsule has a seam due to the fusing step.

The seamed soft capsule shell of the present invention contains various types of gelatin and plasticizer. The gelatins include those derived from animals such as cattle and swine. The examples of type of gelatin include an alkali-treated gelatin, an acid-treated gelatin, and a chemically modified gelatin. The gelatin may be used alone or as a mixture of two or more.

The alkali-treated gelatins are those obtained by hydrolyzing a raw material of gelatin such as collagen or ossein with an alkaline material such as a lime solution and then extracting the hydrolyzate, while the acid-treated gelatins are those obtained by hydrolyzing a collagen with an acidic material such as dilute hydrochloric acid or dilute sulfuric acid. The chemically modified gelatins include, but not limited to, those prepared by reacting an amino group of a gelatin with succinic acid, phthalic acid, acetic acid or the like. The alkali-treated or acid-treated gelatins may be used as a starting material to prepare the chemically modified gelatins.

Suitable plasticizers include concentrated glycerin, sorbitol, maltose, glucose, maltitose, sucrose, xylitol, mannitol, erythritol, polyethylene glycols having a molecular weight of 400-6000, etc.

The seamed soft capsule shell of the present invention preferably contains both gelatin and concentrated glycerin.

When concentrated glycerin is used as a plasticizer, it is preferably combined with gelatin at a ratio of 15-50 parts by weight, more preferably 20-45 parts by weight to 100 parts by weight of the gelatin.

The seamed soft capsule shell of the present invention may contain further plasticizers such as sorbitol, maltose, glucose, maltitose, sucrose, xylitol, mannitol and erythritol. In the present invention, these are added to suppress the deterioration, such as inteneration, stickiness and disintegration delay, of the shell. Sorbitol is preferred among all. Preferably, sorbitol is present in the capsule shell at a ratio of 5-15 parts by weight, more preferably 7-10 parts by weight to 100 parts by weight of gelatin.

The seamed soft capsule shell preferably has a thickness of 28-40 x 10⁻³ inch (71-100 x 10⁻² mm), more preferably 30-35 x 10⁻³ inch (76-89 x 10⁻² mm) as formed. The term "thickness as formed" is regarded as the thickness of a gelatin sheet out of which the shells are stamped. The term "thickness as formed" is hereinafter referred to as "thickness".

The examples of the formulation of the present invention include those in which a solution of BO-653 in a fat and oil, preferably a vegetable oil, more preferably soybean oil, containing unsaturated fatty acids as major constituent is filled into a seamed soft capsule, the shell of which preferably contains gelatin, concentrated glycerin and sorbitol.

The drug substance used for preparing the formulation of the present invention can be produced by known method. The compounds represented by general formula (1) including BO-653 can be produced by the method described for example in WO94/08930. Probucol can also be prepared by known method.

Fat and oils used for dissolving the drug substance therein, and the gelatins and plasticizers described above which are main components of the seamed soft capsule shells are all commercially available.

The formulations of the present invention can be prepared by known method. For example, the present formulations may be prepared via the steps of: preparing a filling solution, preparing capsule shells, forming a seamed soft capsule by encapsulating the filling solution with the shells, and drying the formed capsule.

The step of preparing the filling solution comprises mixing a drug substance and a fat and oil. The step of preparing capsule shells comprises mixing a gelatin, a plasticizer and the like.

The step of forming a seamed soft capsule is carried out by the rotary process. The rotary process comprises: (1) passing a continuous gelatin sheet between two rotating rollers opposed to each other and stamping capsule shells out of the gelatin sheet by the action of rollers; and (2) at the same time as the stamping, injecting the filling solution into a pair of the capsule shells and then fusing the edges of the shells by heat action to form a seamed soft capsule.

The drying step comprises drying the seamed soft capsule at about 25°C in the air controlled at a relative air humidity of about 5-10%.

The invention will be illustrated in more detail with reference to the following Examples, but the present invention is not deemed to be limited thereto. Test results demonstrating the utility of the present invention will also be shown.

### EXAMPLES

### Example 1: Preparation of seamed soft capsule formulation containing BO-653

Seamed soft capsule formulations 1-4 below were prepared by the method described above.

### Formulation 1

Filling solution: 10% w/w solution of BO-653 in soybean oil.
Capsule shells: alkali-treated gelatin/concentrated glycerin = 100/30 w/w.

### Formulation 2

Filling solution: 10% w/w solution of BO-653 in MCT.
Capsule shells: alkali-treated gelatin/concentrated glycerin = 100/30 w/w.

### Formulation 3

Filling solution: 50% w/w solution of BO-653 in soybean oil.
Capsule shells: as shown in Table 1.

**Table 1:**

| Capsule shells | | | |
|---|---|---|---|
| Capsule No. | Thickness | | Content of concentrated glycerin (per 100 parts by weight of gelatin) |
| | x 10⁻³ inch | x 10⁻² mm | |
| 1 | 26 | 66 | 30.0 |
| 2 | 30 | 76 | 30.0 |
| 3 | 34 | 86 | 30.0 |
| 4 | 26 | 66 | 44.2 |
| 5 | 30 | 76 | 44.2 |
| 6 | 34 | 86 | 44.2 |
| 7 | 26 | 66 | 56.4 |
| 8 | 30 | 76 | 56.4 |
| 9 | 34 | 86 | 56.4 |
| Dosage form: 5 oval Gelatin: alkali-treated gelatin Amount of solution filled: 250 mg/capsule. | | | |

### Formulation 4

Filling solution: 4, 40 and 80% w/w solutions of BO-653 in soybean oil.
Capsule shells: as shown in Table 2.

**Table 2**

| | |
|---|---|
| Content of concentrated glycerin | 20 (per 100 parts by weight of gelatin) |
| Content of sorbitol | 7 (per 100 parts by weight of gelatin) |
| Thickness | 30 x 10⁻³ inch (76 x 10⁻²mm) |
| Gelatin: alkali-treated gelatin. | |

### Test example 1: Stability of BO-653 in various fat and oils

The influence of oxygen on the stability of BO-653 in various fat and oils was tested. BO-653 was dissolved in soybean oil, olive oil, MCT, tricaprylin, and linoleic acid separately to give a concentration of 50% by weight. 70 mg of each of the solutions was placed into individual 5 cc glass bottles. Separately, 35 mg of BO-653 was directly placed into a 5 cc glass bottle. The samples were prepared in duplicate. One set of samples were subjected to an argon replacement of the headspace of the bottles and then tightly sealed, while the other set of samples were sealed without argon replacement. Accelerated test at 80°C was conducted using all of the samples. The residual ratio of BO-653 in each bottle after 17 hours was determined by HPLC under the conditions shown in Table 3.

**Table 3:**

| HPLC analysis | |
|---|---|
| Column | DEVELOSIL ODS-HG-5,250 mm x 4.6 mm I.D. (Nomura Chemical) |
| Mobile phase | Acetonitrile/2-propanol/water (800:120:80) |
| Flow rate | 1 mL/min |
| Column temperature | Room temperature |
| Detection wavelength | 300 nm |

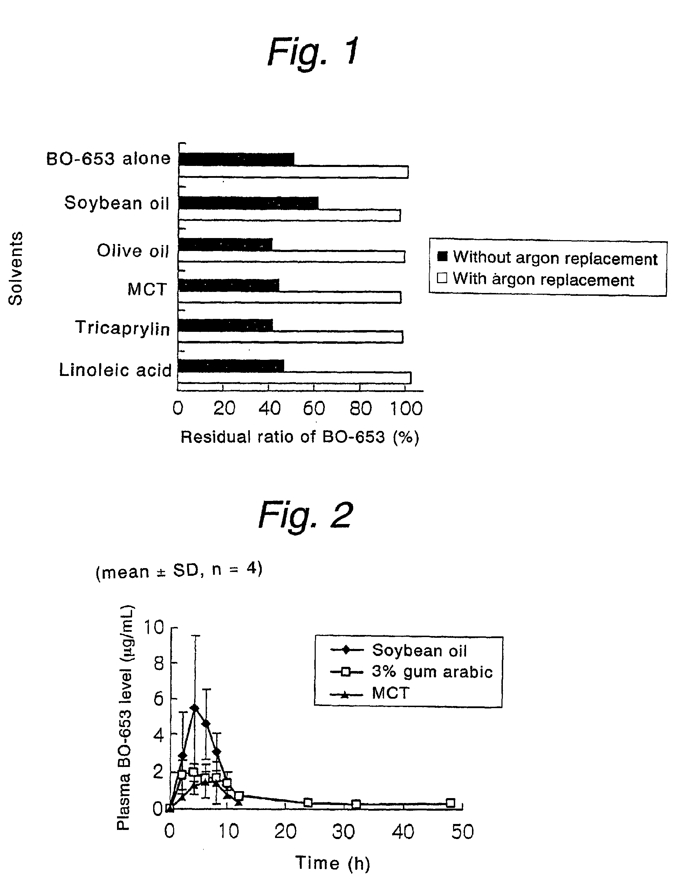

The test results are shown in Fig. 1.

For the samples, including one containing only BO-653, with the argon replacement (i.e, in the absence of oxygen), little loss of BO-653 was observed indicating that BO-653 is stable in any fat and oils used. For the samples without the argon replacement (i.e, in the presence of oxygen), the stability of BO-653 varied depending on the fat and oils used. Relatively high stability was observed in the case where soybean oil was used.

### Test example 2: Influence of administration solvents on the capability of being absorbed by body of RO-653

In order to determine the influence of administration solvents on the capability of being absorbed by body of BO-653, the following tests were conducted using the samples prepared with different solvents shown in Table 4.

**Table 4:**

| Samples | |
|---|---|
| Sample 1 | BO-653 solution in soybean oil having a concentration of 25 mg/mL |
| Sample 2 | BO-653 solution in MCT having a concentration of 25 mg/mL |
| Sample 3 | BO-653 suspension in 3% aqueous solution of gum arabic having a BO-653 concentration of 25 mg/mL |

8-week old SD male rats (270-300 g) were divided into several groups of four. Each of the samples was orally administered via a stomach catheter to the animals under fasting so that a dose of BO-653 of 50 mg/kg was given. After a certain period, blood was collected from the tail vein, and plasma was separated and then measured for plasma BO-653 levels by HPLC under conditions shown in Table 5.

**Table 5:**

| HPLC analysis | |
|---|---|
| Column | TSK-gel ODS-80Ts, 2.0 mm x 150 mm (TOSOH) |
| Mobile phase | Methanol/water (95:5) |
| Flow rate | 200 µL/min |
| Column temperature | Room temperature |
| Detection wavelength | 300 nm |

The test results are shown in Table 6 and Fig. 2.

**Table 6:**

| Pharmacokinetic parameters (mean ± SD, n = 4) | | |
|---|---|---|
| Sample | Cₘₐₓ in µg/mL | AUC₀₋₄₈ₕ in µg.h/mL |
| Sample 1 | 6.1 ± 3.5 | 44.6 ± 15.7 |
| Sample 2 | 2.1 ± 0.5 | 10.6 ± 4.3 |
| Sample 3 | 2.2 ± 0.3 | 23.2 ± 7.3 |

In Table 6, Cₘₐₓ represents the peak plasma level and AUC₀₋₄₈ₕ represents the area under the plasma level-time curves shown in Fig. 2 from 0 to 48 hours after administration. In Fig. 2, "soybean oil", "MCT" and "3% gum arabic" are for samples 1, 2 and 3, respectively. Sample 1 in which soybean oil was used as solvent showed the highest plasma level shift and the AUC value for Sample 1 was about 4 times and about twice as high as compared with those for samples 2 and 3, respectively.

### Test example 3: Stability of seamed soft capsule formulation of BO-653 (1)

Formulations 1 and 2 obtained in Example 1 were tested for their stability under the conditions below: Condition 1:
Formulation 1 was left at 40°C in a 100% oxygen atmosphere. The content of BO-653 after 1 month was determined by HPLC under the conditions shown in Table 3. Condition 2:
Formulation 1 was left at 50°C in a 100% oxygen atmosphere. The content of BO-653 after 2 weeks was determined by HPLC under the conditions shown in Table 3. Condition 3:
Formulation 2 was left at 50°C in a 100% oxygen atmosphere. The content of BO-653 after 3 weeks was determined by HPLC under the conditions shown in Table 3.

As a control, BO-653 was dissolved in soybean oil to give a concentration of 10% by weight and the solution was left at 40°C in a 100% oxygen atmosphere in the form that the solution was not filled into a soft capsule. The residual ratio of BO-653 after 1 month was determined by HPLC under the conditions shown in Table 3. The test results are shown in Table 7.

**Table 7:**

| Residual ratio (%) of BO-653 after various accelerated tests | |
|---|---|
| Condition 1 | 100.7 |
| Control | < 0.2 |
| Condition 2 | 99.6 |
| Condition 3 | 92.8 |

As apparent from comparison between condition 1 and control, BO-653 shows very high stability when it is filled into a soft capsule. Comparison between condition 2 and condition 3 shows that the stability of BO-653 in a soft capsule is influenced by the fat and oil used and that the stability is especially high when BO-653 is dissolved in soybean oil.

### Test example 4: Stability of seamed soft capsule formulations of BO-653 (2)

Formulation 3 obtained in Example 1 was stored at 50°C in a 100% oxygen atmosphere. The residual ratio of BO-653 after 1 month was determined by HPLC under the conditions shown in Table 3. The test results are shown in Table 8.

**Table 8:**

| Content (%) of BO-653 after left at 50°C in a 100% oxygen atmosphere for 1 month | | | |
|---|---|---|---|
| Glycerin content | Thickness | | |
| | 26 x 10⁻³ inch (66 x 10⁻² mm) | 30 x 10⁻³ inch (76 x 10⁻² mm) | 34 x 10⁻³ inch (86 x 10⁻² mm) |
| 30.0 | 99.4 | 99.9 | 99.7 |
| 44.2 | 98.8 | 100.0 | 100.8 |
| 56.4 | -^{*1} | 97.0 | 98.8 |

| | | | |
|---|---|---|---|
| *1 Not evaluated because the capsule shell melted. | | | |

BO-653 shows especially high stability when encapsulated in a soft capsule shell having a thickness of 30-34 x 10⁻³ inch (76-86 x 10⁻² mm) and containing 30.0-44.2 parts by weight of concentrated glycerin per 100 parts by weight of gelatin. Especially preferred thickness is 30-34 x 10⁻³ inch (76-86 x 10⁻² mm) because it allows the shell to have an excellent oxygen barrier property and a good disintegrating property. In order to improve the oxygen barrier property, the shell preferably contains 30 parts by weight of concentrated glycerin per 100 parts by weight of gelatin. Seamed soft capsule formulation 3 is considered to show an excellent stability and a good disintegrating property.

### Test example 5: Stability of seamed soft capsule formulations of BO-653 (3)

Formulation 4 obtained in Example 1 was stored under various conditions shown in Table 9. The residual ratio of BO-653 after the storage was determined by HPLC under the conditions shown in Table 3. The test results are shown in Table 9.

**Table 9:**

| Residual ratio (%) of BO-653 after various accelerated tests | | | |
|---|---|---|---|
| Accelerated test conditions | BO-653 content in filling solution | | |
| | 4% by weight | 40% by weight | 80% by weight |
| 40°C, RH75% - 1M | 99.0 | 100.1 | 99.8 |
| 50°C, 100% oxygen atmosphere-1M | 100.0 | 100.7 | 101.0 |
| Irradiation with 1,200,000 lux.hrs white light | 100.0 | 100.0 | 99.5 |
| Irradiation with 1,200,000 lux.hrs white light followed by 400 W.hrs/m² UV rays | 98.0 | 99.7 | 99.8 |

These formulations show an excellent stability under any storage conditions or at any BO-653 contents. The shells were neither intenerated nor sticky. It can be concluded from these results that Formulation 4, the shell of which contains sorbitol as a further component and has a lower glycerin content than that of Formulation 3, is more preferred seamed soft capsule formulation than any other formulations because it not only exhibits an excellent stability but also suppresses inteneration, stickiness, and disintegration delay of the shell.

### INDUSTRIAL APPLICABILITY

The present invention provides BO-653 formulation having an excellent stability and a capability of being enterally absorbed.

## Claims

1. A seamed soft capsule formulation in which a solution of a dihydrobenzofuran derivative represented by general formula (1): 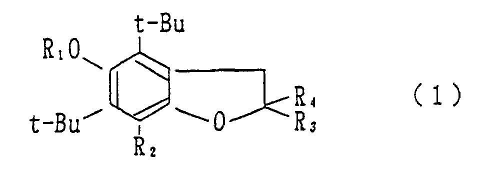 wherein R₁ represents a hydrogen atom or an acyl group; R₂ represents a hydrogen atom or a lower alkyl group; and R₃ and R₄, which may be identical or different, represent a hydrogen atom or a substituted or unsubstituted alkyl, alkenyl, alkynyl or aryl group, or probucol in a fat and oil is filled into a seamed soft capsule.

2. The formulation of Claim 1 wherein said compound represented by general formula (1) is 4,6-di-tert-butyl-2,2-di-n-pentyl-5-hydroxy-2,3-dihydrobenzofuran.

3. The formulation of Claim 1 or 2 wherein said fat and oil is a vegetable oil.

4. The formulation of Claim 3 wherein said vegetable oil contains an unsaturated fatty acid as a major constituent.

5. The formulation of Claim 4 wherein said vegetable oil is soybean oil.

6. The formulation of Claim 1 or 2 wherein shell of said soft capsule contains gelatin and concentrated glycerin.

7. The formulation of Claim 1 or 2 wherein shell of said soft capsule contains sorbitol.

8. The formulation of Claim 6 wherein said shell of said soft capsule further contains sorbitol.

9. The formulation of Claim 8 wherein said soft capsule contains 15-50 parts by weight of concentrated glycerin per 100 parts by weight of gelatin.

10. The formulation of Claim 9 wherein said soft capsule contains 20-45 parts by weight of concentrated glycerin per 100 parts by weight of gelatin.

11. The formulation of Claim 8 wherein said shell of said soft capsule contains 5-15 parts by weight of sorbitol per 100 parts by weight of gelatin.

12. The formulation of Claim 11 wherein said sorbitol is present in said shell at a ratio of 7-10 parts by weight of sorbitol to 100 parts by weight of gelatin.

13. The formulation of Claim 1 or 2 wherein a thickness as formed of shell of said soft capsule is from 28 x 10⁻³ inch (71 x 10⁻² mm) to 40 x 10⁻³ inch (100 x 10⁻² mm).

14. The formulation of Claim 13 wherein said thickness is from 30 x 10⁻³ inch (76 x 10⁻² mm) to 35 x 10⁻³ in (89 x 10⁻² mm).

15. A seamed soft capsule formulation containing 4,6-di-tert-butyl-2,2-di-n-pentyl-5-hydroxy-2,3-dihydrobenzofuran.

16. A method for treating an arteriosclerosis comprising administering said seamed soft capsule formulation of Claim 1 or 2 to a patient suffering from said arteriosclerosis.

17. A method for treating an arteriosclerosis comprising administering said seamed soft capsule formulation of Claim 15 to a patient suffering from said arteriosclerosis.
